# EUROPEAN PATENT SPECIFICATION

(11) **EP 3 030 207 B1**
(45) Date of publication and mention of the grant of the patent: **25.12.2019**
(21) Application number: 14752524.0
(22) Date of filing: 31.07.2014
(51) Int. Cl.: A61F 13/02, A61F 13/00, A61F 5/34

(54) **A SUPPORT DEVICE WITH A CONTAINED CUSHIONING ELEMENT**
TRÄGERVORRICHTUNG MIT EINEM DÄMPFUNGSELEMENT
DISPOSITIF DE SUPPORT AYANT UN ÉLÉMENT D'AMORTISSEMENT CONTENU

(30) Priority: 05.08.2013 US 201361862143 P
(43) Date of publication of application: 15.06.2016
(73) Proprietor: 3M Innovative Properties Company, St. Paul, MN 55133-3427 (US)
(72) Inventor: KARIM, Naimul, Saint Paul, Minnesota 55133-3427 (US); EISENBERG, Peter, M., Saint Paul, Minnesota 55133-3427 (US); HU, Jia, Saint Paul, Minnesota 55133-3427 (US); HANSON, Jennifer, N., Saint Paul, Minnesota 55133-3427 (US); SANGHI, Shilpi, K., Saint Paul, Minnesota 55133-3427 (US)
(74) Representative: Vossius & Partner Patentanwälte Rechtsanwälte mbB
(86) International application number: PCT/US2014/049148
(87) International publication number: WO 2015/020875

(56) References cited:
- WO-A1-93/19710
- JP-A- H1 085 253
- US-A- 5 244 457
- US-A1- 2004 126 413

## Description

### Field

The present disclosure relates to a support device with a contained cushioning element.

### Background

In a variety of applications, medical devices, such as tubing, catheters, and monitoring devices, must be placed directly adjacent to skin. These medical devices must be secured to the skin, typically through tapes, adhesives, or rigid securement devices. The medical devices and securement devices are typically rigid and can cause discomfort, irritation, and a pressure ulcer on the skin.

It is desirable to keep the area containing the medical device clean. Transparent film dressings are widely used as protective layers over wounds or medical device insertion sites to provide a barrier to contaminating liquids and bacteria. However, the transparent film alone may not give sufficient comfort to an overlying medical device.

Wound dressings can include foam material secured to a transparent film. However, these foam materials are highly absorbent of fluid from the skin, wound, or environment, which requires changing the wound dressing when the foam becomes saturated. In some instances, it is desirable to introduce fluid to the area for cleaning, but undesirable for that fluid to be absorbed within the dressing.

US 2004/0126413 A1 discloses a wound dressing including an absorbent core defining opposed proximal and distal surfaces. A perforated skin adherent facing layer is secured to a proximal surface of the absorbent core and a liquid impervious, vapor permeable backing layer is connected to a distal surface of the absorbent core. The backing layer defines at least one compliant element disassociated from the distal surface of the absorbent core and including at least one ridge extending outwardly relative to the distal surface of the absorbent core.

US 5 244 457 A discloses a vented dressing comprising a thin conformable vapor-permeable, liquid-impermeable sheet material having a generally centrally disposed wound-covering portion for placement directly over the wound and a peripheral portion defining the central portion and which is intended to be secured to the whole skin surrounding the wound.

WO 93/19710 A1 discloses an absorbent wound dressing which comprises a layer of hydrophobic silicone gel intended to lie against a wound surface when the dressing is worn, a layer of carrier material which carries the gel layer and affords the requisite strength thereto, and an absorbent body which is placed on that side of the carrier material and gel layer which lie distal from the wound surface in use. The carrier material and the gel layer have mutually coinciding penetrating perforations at least within the region of the absorbent body.

JP H10 85253 A discloses a support for a medical instrument.

### Summary

A support device as recited in the independent claim is provided. The dependent claims define embodiments.

The disclosed support device includes a contained cushioning element, which allows for cleaning of the support device but limits absorption of fluid into the cushioning element.

In one embodiment, the support device comprises a backing layer, a cushioning element, and a base layer. The backing layer comprises a first surface and second surface, opposite the first surface, wherein the backing layer is highly moisture vapor permeable and the first surface of the backing layer is liquid water impermeable. The cushioning element is positioned adjacent the second surface of the backing layer. The base layer comprises a first surface adjacent the cushioning element and a second surface, opposite the first surface of the cushioning element. The base layer is highly moisture vapor permeable. The base layer is entirely contiguous. The second surface of the base layer comprises an adhesive. The base layer and backing layer connect entirely around the cushioning element.

In one embodiment, the backing layer or base layer each comprises a plurality of layers of material. In one embodiment, the backing layer comprises a film. In one embodiment, the second surface of the backing layer at least partially comprises an adhesive for connecting with the base layer. In one embodiment, an area of the backing layer and base layer is larger than an area of the cushioning element, such that the backing layer and base layer entirely extend beyond the area of the cushioning element.

In one embodiment, the cushioning element has a thickness substantially greater than a thickness of the backing layer and base layer. In one embodiment, the cushioning element is deformable and compressible. In one embodiment, the cushioning element comprises a foam, sponge, gel, hydrocolloid, fabric of a lofty nonwoven, woven, or knitted material. In one embodiment, the cushioning element is a nonabsorbent. In one embodiment, the cushioning element is highly moisture vapor permeable.

In one embodiment, the base layer is entirely contiguous such that the base layer is entirely of continuous and uniform material construction. In one embodiment, the base layer is entirely contiguous such that the base layer is of continuous and uniform material construction. In one embodiment, the base layer is entirely contiguous such that the base layer is free of perforations. In one embodiment, the base layer is a film, woven fabric, knitted fabric, or nonwoven. In one embodiment, the base layer is liquid water impermeable.

In one embodiment, the adhesive secures to skin. In one embodiment, the support device further comprising a securement device secured to the first surface of the backing layer, adjacent the cushioning element. In one embodiment, the securement device holds a medical device.

The words "entirely contiguous" as used herein means that the surface area is of substantially continuous material construction and is substantially free of large voids, gaps, or perforations in the material construction. On a small scale, a material may still be entirely contiguous even if there are gaps in the material. For example, films, nonwovens, paper, and fabrics could all be formed in a way such that it is entirely contiguous.

The words "highly moisture vapor permeable" mean that the material transmits moisture vapor at a rate similar to or greater than human skin. For example, using the inverted cup method as described in U.S. Pat. No. 4,595,001, highly moisture vapor permeable means having a rate of at least 300 g/m² /24 hrs at 37°C/100-10% RH.

The words "liquid water impermeable" mean that if liquid water is put in direct contact with one surface of the material then, under normal atmospheric pressure, it is not readily transported to the opposite surface of the material.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments.

As used herein, "a," "an," "the," "at least one," and "one or more" are used interchangeably. The term "and/or" (if used) means one or all of the identified elements or a combination of any two or more of the identified elements.

### Brief Description of the Drawings

FIG. 1 is a perspective view of first embodiment of a support device;
FIG. 2 is a side view of the support device of FIG. 1;
FIG. 3 is a bottom view of the support device of FIGS. 1-2;
FIG. 4 is a side view of the support device of FIGS. 1-3 with an applied medical device.

While the above-identified drawings and figures set forth embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of this invention.
The figures may not be drawn to scale.

### Detailed Description

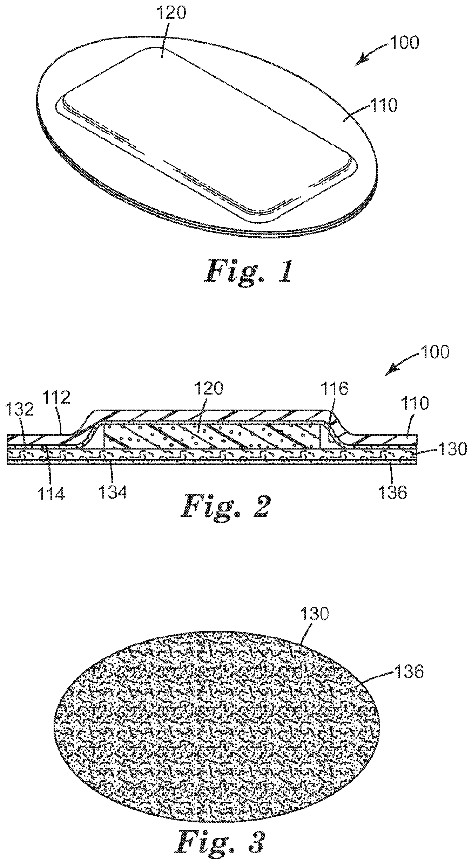

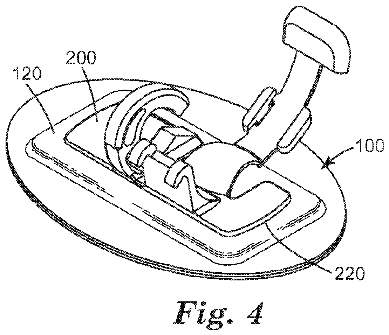

FIG. 1 is a perspective view of first embodiment of a support device 100. FIG. 2 is a side view of the support device 100, and FIG. 3 is a bottom view of the support device 100. FIG. 4 is a side view of the support device 100 of FIGS. 1-3 with an applied medical device 200.

The support device 100 comprises a backing layer 110, a base layer 130, and a cushioning element 120 positioned between the backing layer 110 and base layer 130.

The backing layer 110 comprises a first surface 112 and second surface 114, opposite the first surface. The first surface 112 of the backing layer 110 typically faces away from the surface to which the support device 100 attaches. Also, the first surface 112 is the surface that contacts with the applied medical device 200. The backing layer 110 is highly moisture vapor permeable and the first surface 112 of the backing layer 110 is liquid water impermeable.

The cushioning element 120 is placed adjacent the backing layer 110 and base layer 130. The cushioning element 120 provides comfort and helps prevent an overlying medical device 200 from irritating or damaging the underlying surface. Therefore, typically the cushioning element 120 is deformable and compressible. In one embodiment, the cushioning element 120 has a thickness substantially greater than a thickness of the backing layer 110 and base layer 130. In one embodiment, the cushioning element 120 is nonabsorbent. 10. In one embodiment, the cushioning element 120 is highly moisture vapor permeable. In one embodiment, the cushioning element can distribute pressure.

The base layer 130 comprises a first surface 132 adjacent the cushioning element 120 and a second surface 134, opposite the first surface 132 of the cushioning element 120. The second surface 134 of the base layer 130 comprises an adhesive 136. In one embodiment, substantially the entire second surface 134 comprises the adhesive 136. In one embodiment, the adhesive 136 is on a portion of the second surface 134. For example, the adhesive 136 may be pattern coated to the second surface 134 of the base layer 130.

The base layer 130 is entirely contiguous. In one embodiment, the base layer 130 is of continuous and uniform material construction. In one embodiment, the base layer 130 is of substantial uniform thickness across the entire base layer 130. The base layer 130 is highly moisture vapor permeable. In one embodiment, the base layer 130 is liquid water impermeable.

The base layer 130 and backing layer 110 can each comprise one or more layers of materials. In one embodiment, the base layer 130 and backing layer 110 are of substantially the same area. In one embodiment the base layer 130 and backing layer 110 have a larger surface area than the surface area of the cushioning element 120.

To contain the cushioning element 120, the base layer 130 and backing layer 110 connect entirely around the cushioning element 120, such as can best be seen in FIG. 1 or FIG. 2. In one embodiment, base layer 130, backing layer 110 or both the base layer 130 and backing layer 110 comprise a securing adhesive 116 for connecting the base layer 130 and backing layer 110.

In the described construction, the cushioning element 120 is entirely contained within structure of the backing layer 110 and base layer 130. The base layer 130 includes an adhesive 136 for securing the support device 100 to a surface, such as skin. The backing layer 110 includes a first surface 112 that is liquid water impermeable, which allows for easy cleaning of the support device 100. Overall, in one embodiment, the backing layer 110, cushioning element 120, and base layer 130 are highly moisture vapor permeable, making the support device well suited for application to skin. Distinguishing from a wound dressing, the support device 100 includes the base layer 130 that is entirely contiguous, which provides significant containment of the cushioning element from liquid.

In one embodiment, the support device 100 can be applied to skin to protect the underlying skin from contact with hard, abrading, or irritating surface. In one embodiment, the support device 100 can be applied to skin to protect the skin from applied pressure, which may result in a pressure ulcer on the skin. With the liquid water impermeable backing layer 110, the support device 100 can be easily cleaned.

The support device 100 is well suited for application and adhering to skin and can be cleaned without the liquid water penetrating into the cushioning element. Limiting absorption of liquid into the cushioning element 120 increases the time that the support device 100 can be worn on skin without a need for removal and changing.

As discussed above, a device 200, such as a medical device can be applied to the first surface 112 of the backing layer 110, adjacent the cushioning element 120. The device 200 can be permanently or removably secured to the first surface 112. For example, tape, hook/loop, or adhesives can be used to secure the device 200 to the support device 100. Examples of medical devices include tubing, catheters, ports, or securement devices for securing tubing or catheters. In one embodiment, a securement device 200 such as shown in FIG. 4 and described in US Patent Application 61/862,284 filed August 5, 2013 and published as US 2015/0038912 A1, is permanently secured to the first surface 112 of the backing layer 110, and the securement device 200 is used to secure other medical devices, such as tubing or a catheter.

### Backing Layer

The backing layer can provide an impermeable barrier to the passage of liquids and at least some gases. Representative barriers may include non-woven and woven fibrous webs, knits, films, foams, polymeric films and other familiar backing materials. In some embodiments, a transparent substrate is desirable to allow for viewing of the underlying skin.

In one embodiment, the backing layer has high moisture vapor permeability, but generally impermeable to liquid water so that microbes and other contaminants are sealed out from the area under the substrate. One example of a suitable material is a high moisture vapor permeable film such as described in US Patents 3,645,835 and 4,595,001. In high moisture vapor permeable film/adhesive composites, the composite should transmit moisture vapor at a rate equal to or greater than human skin, in embodiments at a rate of at least 300 g/m² /24 hrs at 37°C/100-10% RH; or in one embodiment at a rate of at least 700 g/m² /24 hrs at 37°C/100-10% RH; or in one embodiment at a rate of at least 2000 g/m² /24 hrs at 37°C/100-10% RH using the inverted cup method as described in U.S. Pat. No. 4,595,001. If more than one layer is used, perforated substrates or films or pattern coated adhesives may be used to increase the moisture vapor transmission, but overall the backing layer is liquid water impermeable. In one embodiment, the backing layer is an elastomeric polyurethane, polyester, or polyether block amide film. These films combine the desirable properties of resiliency, elasticity, high moisture vapor permeability, and transparency. A description of this characteristic of backing layers can be found in issued U.S. Patent Nos. 5,088,483 and 5,160,315.

Commercially available examples of potentially suitable backing materials may include the thin polymeric film backings sold under the trade names TEGADERM (3M Company), OPSITE (Smith & Nephew), etc. Many other backings may also be used, including those commonly used in the manufacture of surgical incise drapes (e.g., incise drapes manufactured by 3M Company under the trade names STERIDRAPE and IOBAN), etc.

Because fluids may be actively removed from the sealed environments defined by the medical dressings, a relatively high moisture vapor permeable backing may not be required. As a result, some other potentially useful backing materials may include, e.g., metallocene polyolefins and SBS and SIS block copolymer materials.

Regardless, however, it may be desirable that the backing layer be kept relatively thin to, e.g., improve conformability. For example, the backing layer may be formed of polymeric films with a thickness of 200 micrometers or less, or 100 micrometers or less, potentially 50 micrometers or less, or even 25 micrometers or less.

### Cushioning Element

The cushioning element provides comfort and therefore typically is deformable and compressible. Suitable materials include a foam, sponge, gel, hydrocolloid, nonwoven, woven, or knitted material. In one embodiment the cushioning element is nonabsorbent.

### Base Layer

The base layer provides a surface to which the adhesive 136 is applied to and also provides a surface that contains the cushioning element.

In one embodiment, the base layer can be of a construction substantially as described above for the backing layer and therefore includes non-woven and woven fibrous webs, knits, films, foams polymeric films and other familiar backing materials.

The base layer has high moisture vapor permeability. In one embodiment, the base layer is impermeable to liquid water. To limit introduction of liquid into the cushioning element, the base layer is not perforated.

Commercially available examples of potentially suitable base layer materials may include SONTARA brand fabric (DuPont). Regardless, however, it may be desirable that the base layer be kept relatively thin to, e.g., improve conformability. For example, the base layer may be formed of polymeric films with a thickness of 200 micrometers or less, or 100 micrometers or less, potentially 50 micrometers or less, or even 25 micrometers or less. The base layer can also be comprised of a combination of two materials, such as, for example, two films (coextruded), film and fabric (woven, knitted, nonwoven).

### Adhesive

Suitable adhesive for use on the second surface 134 of the base layer 130 for securing the support device 100 to a surface include adhesive that provides acceptable adhesion to skin and is acceptable for use on skin (e.g., the adhesive should preferably be non-irritating and non-sensitizing). Suitable adhesives are pressure sensitive and in certain embodiments have a relatively high moisture vapor transmission rate to allow for moisture evaporation. Suitable pressure sensitive adhesives include those based on acrylates, urethane, hydrogels, hydrocolloids, block copolymers, silicones, rubber based adhesives (including natural rubber, polyisoprene, polyisobutylene, butyl rubber etc.) as well as combinations of these adhesives. The adhesive component may contain tackifiers, plasticizers, rheology modifiers as well as active components including for example an antimicrobial agent.

The pressure sensitive adhesives that may be used in the wound dressings may include adhesives that are typically applied to the skin such as the acrylate copolymers described in U.S. Patent No. RE 24,906, particularly a 97:3 isooctyl acrylate:acrylamide copolymer. Another example may include a 70:15:15 isooctyl acrylate: ethyleneoxide acrylate:acrylic acid terpolymer, as described in U.S. Patent No. 4,737,410 (Example 31). Other potentially useful adhesives are described in U.S. Patent Nos. 3,389,827; 4,112,213; 4,310,509; and 4,323,557. Inclusion of medicaments or antimicrobial agents in the adhesive is also contemplated, as described in U.S. Patent Nos. 4,310,509 and 4,323,557.

Silicone adhesive can also be used. Generally, silicone adhesives can provide suitable adhesion to skin while gently removing from skin. Suitable silicone adhesives are disclosed in PCT Publications WO2010/056541 and WO2010/056543.

The pressure sensitive adhesives may, in some embodiments, transmit moisture vapor at a rate greater to or equal to that of human skin. While such a characteristic can be achieved through the selection of an appropriate adhesive, it is also contemplated that other methods of achieving a high relative rate of moisture vapor transmission may be used, such as pattern coating the adhesive on the backing, as described in U.S. Patent No. 4,595,001. Other potentially suitable pressure sensitive adhesives may include blown-micro-fiber (BMF) adhesives such as, for example, those described in U.S. Patent No. 6,994,904. The pressure sensitive adhesive used in the wound dressing may also include one or more areas in which the adhesive itself includes structures such as, e.g., the microreplicated structures described in U.S. Patent No. 6,893,655.

Issued U.S. Patent Nos. 3,645,835 and 4,595,001 describe methods of making such films and methods for testing their permeability. Preferably, the film/adhesive composite should transmit moisture vapor at a rate equal to or greater than human skin. Preferably, the adhesive coated material transmits moisture vapor at a rate of at least 300 g/m² /24 hrs at 37°C/100-10% RH; or in one embodiment at a rate of at least 700 g/m² /24 hrs at 37°C/100-10% RH; or in one embodiment at a rate of at least 2000 g/m² /24 hrs at 37°C/100-10% RH using the inverted cup method as described in U.S. Pat. No. 4,595,001.

Different portions of the dressing may include different adhesives, such as disclosed in U.S. Patent Application 61/664,246 filed June 26, 2012 titled "Medical Dressing with Multiple Adhesives." For example, a portion may include an acrylate adhesive while another portion may include a silicone adhesive. In one embodiment, to prevent edge separation, adjacent the perimeter is acrylate adhesive, while near the central portion there is silicone adhesive. In one embodiment, to strongly secure with a device or tubing near the central portion there is acrylate adhesive, while near the perimeter in contact with skin is silicone adhesive.

### Optional Components

An optional release liner may be included that covers all or a portion of the adhesives to prevent contamination of the adhesives. In one embodiment, the package that contains the support device 100 may serve as a release liner.

An optional carrier may be included that covers all or a portion of the first surface of the backing layer 110, providing structural support if the dressing is thin and highly flexible. The carrier may be removable from the first major surface 112 once the support device is placed on skin. The carrier can be constructed of a variety of materials such as fabric that are woven or kitted, nonwoven material, papers, or film. In one embodiment, the carrier is along the perimeter of the first surface of the backing layer 110 and is removable from the first major surface 112 similar to the carrier used the 3M Tegaderm™ Transparent Film Dressing, available from 3M Company, St. Paul, MN.

In one embodiment, indicia for use with the support device 100 can be included that includes a representation (e.g., a pictorial representation) of a medical device such that the indicia mimics the overall shape, appearance and/or configuration of the medical device to provide a visual cue for how to couple the medical device over the support device 100. Such indicia can enhance the usability of the support device 100 and overlying medical device of the present disclosure and can minimize operator errors when applying the systems to patients and coupling medical devices to the support device.

In addition, to the representation of the medical device, the indicia can include directional cues, such as arrows, to indicate how the support device and medical device should be oriented relative to another device, structure, or portion of a patient's body. Support devices of the present disclosure can also include such directional cues.

The indicia can include a variety of markings, graphics, or the like, in order to represent a medical article. For example, in some embodiments, the indicia can include a two-dimensional representation of the outline, outer contours, or outer periphery of a medical article. As such, the indicia may be a simplified representation of the medical device, but it will be clear to a user how to orient the medical device relative to the support device, based on the caricature or representation of the medical device provided by the indicia.

Although specific embodiments of this invention have been shown and described herein, it is understood that these embodiments are merely illustrative of the many possible specific arrangements that can be devised in application of the principles of the invention. Numerous and varied other arrangements can be devised in accordance with these principles by those of ordinary skill in the art without departing from the scope of the invention as defined by the claims. Thus, the scope of the present invention should not be limited to the structures described in this application, but only by the structures described by the language of the claims and the equivalents of those structures.

## Claims

1. A support device (100) comprising:
a backing layer (110) comprising a first surface (112) and second surface (114), opposite the first surface (112), wherein the backing layer (110) is highly moisture vapor permeable having a rate of at least 300 g/m² /24 hrs at 37°C/100-10% RH when measured using the inverted cup method described in U.S. Pat. No. 4,595,001 and the first surface (112) of the backing layer (110) is liquid water impermeable;
a cushioning element (120) positioned adjacent the second surface (114) of the backing layer (110);
a base layer (130) comprising a first surface (132) adjacent the cushioning element (120) and a second surface (134), opposite the first surface (132) of the cushioning element (120), wherein the base layer (130) is highly moisture vapor permeable having a rate of at least 300 g/m² /24 hrs at 37°C/100-10% RH when measured using the inverted cup method described in U.S. Pat. No. 4,595,001;
wherein the base layer (130) is entirely contiguous such that the base layer (130) is free of perforations;
wherein the second surface (134) of the base layer (130) comprises an adhesive (136);
wherein the base layer (130) and backing layer (110) connect entirely around the cushioning element (120).

2. The support device of claim 1, wherein the backing layer (110) or base layer (130) comprises a plurality of layers of material.

3. The support device of any one of the preceding claims, wherein the backing layer (110) comprises a film.

4. The support device of any one of the preceding claims, wherein the second surface (114) of the backing layer (110) at least partially comprises an adhesive (116) for connecting with the base layer (130).

5. The support device of any one of the preceding claims, wherein an area of the backing layer (110) and base layer (130) is larger than an area of the cushioning element (120), such that the backing layer (110) and base layer (130) entirely extend beyond the area of the cushioning element (120).

6. The support device of any one of the preceding claims, wherein the cushioning element (120) has a thickness substantially greater than a thickness of the backing layer (110) and base layer (130).

7. The support device of any one of the preceding claims, wherein the cushioning element (120) is deformable and compressible.

8. The support device of any one of the preceding claims, wherein the cushioning element (120) comprises a foam, sponge, gel, hydrocolloid, fabric of a lofty nonwoven, woven, or knitted material.

9. The support device of any one of the preceding claims, wherein the cushioning element (120) is nonabsorbent.

10. The support device of any one of the preceding claims, wherein the cushioning element (120) is highly moisture vapor permeable.

11. The support device of any one of the preceding claims, wherein the base layer (130) is entirely contiguous such that the base layer (130) is of continuous and uniform material construction.

12. The support device of any one of the preceding claims, wherein the base layer (130) is a film, woven fabric, knitted fabric, or nonwoven.

13. The support device of any one of the preceding claims, wherein the base layer (130) is liquid water impermeable.

14. The support device of any one of the preceding claims, further comprising a securement device (200) secured to the first surface (112) of the backing layer (110), adjacent the cushioning element (120).

15. The support device of claim 14, wherein the securement device (200) holds a medical device.

## Patentansprüche

1. Trägervorrichtung (100), aufweisend:
eine Trägerschicht (110) mit einer ersten Oberfläche (112) und einer zweiten Oberfläche (114) gegenüber der ersten Oberfläche (112), wobei die Trägerschicht (110) stark wasserdampfdurchlässig ist, mit einer Rate von mindestens 300 g/m²/24 h bei 37 °C/100-10 % RF, gemessen unter Verwendung des Umgedrehter-Becher-Verfahrens, das im US-Patent Nr. 4,595,001 beschrieben ist, und die erste Oberfläche (112) der Trägerschicht (110) undurchlässig für flüssiges Wasser ist;
ein Dämpfungselement (120), das benachbart der zweiten Oberfläche (114) der Trägerschicht (110) positioniert ist;
eine Basisschicht (130), aufweisend eine erste Oberfläche (132) benachbart zum Dämpfungselement (120) und eine zweite Oberfläche (134) gegenüber der ersten Oberfläche (132) des Dämpfungselements (120), wobei die Basisschicht (130) stark wasserdampfdurchlässig ist, mit einer Rate von mindestens 300 g/m²/24 h bei 37 °C/100-10 % RF, gemessen unter Verwendung des Umgedrehter-Becher-Verfahrens, das im US-Patent Nr. 4,595,001 beschrieben ist;
wobei die Basisschicht (130) vollständig zusammenhängend ist, so dass die Basisschicht (130) frei von Perforationen ist;
wobei die zweite Oberfläche (134) der Basisschicht (130) einen Kleber (136) aufweist;
wobei die Basisschicht (130) und die Trägerschicht (110) vollständig um das Dämpfungselement (120) herum in Verbindung stehen.

2. Trägervorrichtung nach Anspruch 1, wobei die Trägerschicht (110) oder Basisschicht (130) mehrere Materialschichten aufweist.

3. Trägervorrichtung nach einem der vorstehenden Ansprüche, wobei die Trägerschicht (110) eine Folie aufweist.

4. Trägervorrichtung nach einem der vorstehenden Ansprüche, wobei die zweite Oberfläche (114) der Trägerschicht (110) mindestens teilweise einen Kleber (116) zum Verbinden mit der Basisschicht (130) aufweist.

5. Trägervorrichtung nach einem der vorstehenden Ansprüche, wobei eine Fläche der Trägerschicht (110) und der Basisschicht (130) größer als eine Fläche des Dämpfungselements (120) ist, so dass sich die Trägerschicht (110) und die Basisschicht (130) vollständig über die Fläche des Dämpfungselements (120) hinaus erstrecken.

6. Trägervorrichtung nach einem der vorstehenden Ansprüche, wobei das Dämpfungselement (120) eine Dicke aufweist, die im Wesentlichen größer als eine Dicke der Trägerschicht (110) und der Basisschicht (130) ist.

7. Trägervorrichtung nach einem der vorstehenden Ansprüche, wobei das Dämpfungselement (120) verformbar und zusammendrückbar ist.

8. Trägervorrichtung nach einem der vorstehenden Ansprüche, wobei das Dämpfungselement (120) einen Schaum, einen Schwamm, ein Gel, ein Hydrokolloid, einen Stoff aus einem bauschigen Vlies-, gewebtem oder gestricktem Material aufweist.

9. Trägervorrichtung nach einem der vorstehenden Ansprüche, wobei das Dämpfungselement (120) nicht absorbierend ist.

10. Trägervorrichtung nach einem der vorstehenden Ansprüche, wobei das Dämpfungselement (120) stark wasserdampfdurchlässig ist.

11. Trägervorrichtung nach einem der vorstehenden Ansprüche, wobei die Basisschicht (130) vollständig zusammenhängend ist, so dass die Basisschicht (130) von kontinuierlichem und einheitlichem Materialaufbau ist.

12. Trägervorrichtung nach einem der vorstehenden Ansprüche, wobei die Basisschicht (130) eine Folie, ein Gewebe, ein Gestrick oder ein Vlies ist.

13. Trägervorrichtung nach einem der vorstehenden Ansprüche, wobei die Basisschicht (130) undurchlässig für flüssiges Wasser ist.

14. Trägervorrichtung nach einem der vorstehenden Ansprüche, ferner aufweisend eine Befestigungsvorrichtung (200), die an der ersten Oberfläche (112) der Trägerschicht (110) benachbart des Dämpfungselements (120) befestigt ist.

15. Trägervorrichtung nach Anspruch 14, wobei die Befestigungsvorrichtung (200) eine medizinische Vorrichtung hält.

## Revendications

1. Dispositif de support (100), comprenant :
une couche de renfort (110) comprenant une première surface (112) et une seconde surface (114), opposée à la première surface (112), la couche de renfort (110) étant hautement perméable à la vapeur d'eau ayant un taux d'au moins 300 g/m²/24 h à 37 °C/100-10 % d'humidité relative lorsqu'il est mesuré en utilisant la méthode à coupelle inversée décrite dans le brevet des États-Unis n° 4,595,001 et la première surface (112) de la couche de renfort (110) est imperméable à l'eau liquide ;
un élément d'amortissement (120) positionné adjacent à la seconde surface (114) de la couche de renfort (110) ;
une couche de base (130) comprenant une première surface (132) adjacente à l'élément d'amortissement (120) et une seconde surface (134), opposée à la première surface (132) de l'élément d'amortissement (120), la couche de base (130) étant hautement perméable à la vapeur d'eau ayant un taux d'au moins 300 g/m²/24 h à 37 °C/100-10 % d'humidité relative lorsqu'il est mesuré en utilisant la méthode à coupelle inversée décrite dans le brevet des États-Unis n° 4,595,001 ;
dans lequel la couche de base (130) est entièrement contiguë de sorte que la couche de base (130) est exempte de perforations ;
dans lequel la seconde surface (134) de la couche de base (130) comprend un adhésif (136) ;
dans lequel la couche de base (130) et la couche de renfort (110) se connectent entièrement autour de l'élément d'amortissement (120).

2. Dispositif de support selon la revendication 1, dans lequel la couche de renfort (110) ou la couche de base (130) comprend une pluralité de couches de matériau.

3. Dispositif de support selon l'une quelconque des revendications précédentes, dans lequel la couche de renfort (110) comprend un film.

4. Dispositif de support selon l'une quelconque des revendications précédentes, dans lequel la seconde surface (114) de la couche de renfort (110) comprend au moins partiellement un adhésif (116) pour la connexion avec la couche de base (130).

5. Dispositif de support selon l'une quelconque des revendications précédentes, dans lequel une superficie de la couche de renfort (110) et de la couche de base (130) est plus grande qu'une superficie de l'élément d'amortissement (120), de sorte que la couche de renfort (110) et la couche de base (130) s'étendent entièrement au-delà de la superficie de l'élément d'amortissement (120).

6. Dispositif de support selon l'une quelconque des revendications précédentes, dans lequel l'élément d'amortissement (120) a une épaisseur sensiblement supérieure à une épaisseur de la couche de renfort (110) et de la couche de base (130).

7. Dispositif de support selon l'une quelconque des revendications précédentes, dans lequel l'élément d'amortissement (120) est déformable et compressible.

8. Dispositif de support selon l'une quelconque des revendications précédentes, dans lequel l'élément d'amortissement (120) comprend une mousse, une éponge, un gel, un hydrocolloïde, un tissu d'un matériau non tissé, tissé ou tricoté gonflant.

9. Dispositif de support selon l'une quelconque des revendications précédentes, dans lequel l'élément d'amortissement (120) est non absorbant.

10. Dispositif de support selon l'une quelconque des revendications précédentes, dans lequel l'élément d'amortissement (120) est hautement perméable à la vapeur d'eau.

11. Dispositif de support selon l'une quelconque des revendications précédentes, dans lequel la couche de base (130) est entièrement contiguë de sorte que la couche de base (130) soit de construction matérielle continue et uniforme.

12. Dispositif de support selon l'une quelconque des revendications précédentes, dans lequel la couche de base (130) est un film, un tissu tissé, un tricot ou un non-tissé.

13. Dispositif de support selon l'une quelconque des revendications précédentes, dans lequel la couche de base (130) est imperméable à l'eau liquide.

14. Dispositif de support selon l'une quelconque des revendications précédentes, comprenant en outre un dispositif de fixation (200) fixé à la première surface (112) de la couche de renfort (110), adjacent à l'élément d'amortissement (120).

15. Dispositif de support selon la revendication 14, dans lequel le dispositif de fixation (200) maintient un dispositif médical.
